# EUROPEAN PATENT SPECIFICATION

(11) **EP 3 195 903 B1**
(45) Date of publication and mention of the grant of the patent: **31.03.2021**
(21) Application number: 15841875.6
(22) Date of filing: 10.02.2015
(51) Int. Cl.: A61N 7/02

(54) **ULTRASOUND FOCUSING TRANSDUCER AND ULTRASOUND FOCUSING TREATMENT HEAD**
ULTRASCHALLFOKUSSIERUNGSWANDLER UND ULTRASCHALLFOKUSSIERENDER BEHANDLUNGSKOPF
TRANSDUCTEUR DE FOCALISATION D'ULTRASONS ET TÊTE DE TRAITEMENT DE FOCALISATION D'ULTRASONS

(30) Priority: 17.09.2014 CN 201410476432
(43) Date of publication of application: 26.07.2017
(73) Proprietor: Shenzhen PRO HITU Medical Co., Ltd., Shenzhen City (CN)
(72) Inventor: ZHANG, Xueyong, Shenzhen City (CN); QIAO, Xianbo, Shenzhen City (CN); ZHANG, Ji, Shenzhen City (CN)
(74) Representative: Casalonga
(86) International application number: PCT/CN2015/072706
(87) International publication number: WO 2016/041320

(56) References cited:
- EP-A2- 1 062 933
- CN-A- 101 227 860
- CN-A- 101 766 869
- CN-A- 104 225 811
- CN-U- 201 692 527
- CN-U- 201 692 527
- CN-U- 204 106 877
- CN-Y- 2 657 706
- CN-Y- 2 657 706
- CN-Y- 201 369 686
- JP-A- H02 149 263
- JP-A- 2012 239 791
- US-A1- 2009 299 180

## Description

### TECHNICAL FIELD

The present invention relates to the technical field of ultrasonic medical devices and, more particularly, to an ultrasound focusing transducer and an ultrasound focusing treatment head.

### BACKGROUND

Ultrasound treatment has advantages of noninvasiveness, high efficiency, and controllability, which enables tumors to be heated to an effective treatment temperature through an ultrasound focusing technology. The ultrasound focusing transducer can be used in gynecological diseases, liver cancer, breast cancer, pancreatic cancer, kidney cancer, soft tissue tumors and so on.
Patent application publication No. CN201692527U discloses an ultrasound focusing treatment head, comprising a shell, a piezoelectric ceramic wafer accommodated in the shell, a heat pipe arranged below the piezoelectric ceramic wafer and a cooling fin integrally connected to the heat pipe. The ultrasound focusing treatment head makes the heat transfer in an effective way and is of simple structure and convenient operation.

The ultrasound focusing treatment head includes an ultrasound focusing transducer and a probe accommodated in the ultrasound focusing transducer. The probe is typically a cardiac probe. The ultrasound focusing transducer converts the high-frequency electric energy into ultrasonic waves of the same frequency through a piezoelectric ceramic wafer, and achieves the treatment function through the ultrasonic waves. In the meantime, the cardiac probe monitors the whole treatment process during the course of treatment, and transmits the B-mode ultrasound image in real time. Wherein, the ultrasound focusing transducer includes a piezoelectric ceramic wafer and a support for installing and supporting the piezoelectric ceramic wafer, an installing hole for installing a cardiac probe is provided in the support, and a sealed air cavity is formed between the piezoelectric ceramic wafer and the support. Because the piezoelectric ceramic wafer is fixed on the support by gluing, the sealing performance of gluing is poor under the influence of high-frequency vibration of the piezoelectric ceramic wafer, affecting airtightness of the air cavity, resulting in low stability of the ultrasound focusing transducer, and affecting the service life of the ultrasound focusing transducer.

The foregoing is just for the purpose of assisting in the understanding of the technical solution of the present invention and does not mean that the foregoing is acknowledged as prior art.

### SUMMARY

### Technical problem

The invention is defined by the appended independent claim. Preferred embodiments of the invention are illustrated in the dependent claims. The main object of the present invention is to solve the technical problem of poor airtightness of the air cavity between the piezoelectric ceramic wafer and the support of the ultrasound focusing transducer.

### Technical solution

An example provides an ultrasound focusing transducer, the ultrasound focusing transducer comprises a shell, a piezoelectric ceramic wafer accommodated in the shell, a fixed sealing part, and a support having an installing hole; the piezoelectric ceramic wafer is of a spherical segment shape, and is provided with a through hole of a spherical-segment shape which has a circular first side opening and a circular second side opening opposite to each other, wherein the hole size of the first side opening is smaller than that of the second side opening, one end of the piezoelectric ceramic wafer forming the second side opening abuts against one end of the shell, and one end of the piezoelectric ceramic wafer forming the first side opening faces the other end of the shell; the fixed sealing part is annular, installed on the inner surface of the shell, abutting against the piezoelectric ceramic wafer, and is connected to the support in a sealed manner; the support includes a clamping part, the clamping part is used to press and clamp the periphery of the first side opening of the piezoelectric ceramic wafer and fix the support on the piezoelectric ceramic wafer, and the installing hole is communicated with the spherical-segment shaped through hole, and the fixed sealing part, the support and the piezoelectric ceramic wafer cooperatively form a sealed air cavity.

Preferably, the support comprises a connecting cylinder and a core tube, an inner surface of the one end of the connecting cylinder is mounted on an outer surface of one end of the core tube, and the clamping part is formed between the core tube and the connecting cylinder.

Preferably, the connecting cylinder comprises a first cylinder, an annular connecting part, and a second cylinder, the first cylinder has a first end and a second end, the annular connecting part is formed by extending radially inwardly from the second end, the second cylinder is formed by extending from an inner periphery of the annular connecting part along a direction away from the second end; the core tube comprises a tubular body and a ring piece extending radially outwardly from one end of the tubular body; an inner surface of the second cylinder is mounted on an outer surface of the tubular body, and the clamping part is formed between the end of the second cylinder and the ring piece.

Preferably, a first internal thread is formed on an inner surface of the second cylinder, a first external thread is formed on an outer surface of the tubular body, and the second cylinder is connected to the core tube in a sealed manner by engagement of the first internal thread with the first external thread.

Preferably, the piezoelectric ceramic wafer has a protruded first side surface and a recessed second side surface, the piezoelectric ceramic wafer comprises a ceramic region and a plating region other than the ceramic region, the ceramic region is annular and is arranged on the first side surface around a periphery of the first side opening; the material of the support is conductive, an end of the second cylinder abuts against the ceramic region, and the ring piece acting as a negative electrode abuts against the plating region of the second side surface.

Preferably, the ultrasound focusing transducer further comprises a power cable electrically connected to the plating region of the piezoelectric ceramic wafer, the power cable comprises a negative power cable and a positive power cable, the connecting cylinder further comprises an extending part arranged on an outer surface of the first cylinder, the extending part has a cable hole and a first conductive hole, one end of the negative power cable is electrically connected to the first conductive hole, and the other end of the negative power cable passes through the cable hole.

Preferably, the fixed sealing part comprises an inner pressing ring and an annular baffle, the inner pressing ring comprises a ring body and an abutment part extending axially from one end of the ring body, an outer surface of the ring body is fixedly connected to an inner surface of the shell, the annular baffle is fixed to an inner periphery of the body in a sealed manner, and the abutment part abuts against the first side surface of the piezoelectric ceramic wafer.

Preferably, a second external thread is formed on an outer surface of the ring body, and a second internal thread is formed on an inner surface of the shell, the inner pressing ring and the shell are fixedly connected by the engagement of the second external thread with the second internal thread, an inner surface of the annular baffle and an outer surface of the first cylinder are in contact with each other, and fixedly connected together by gluing in a sealed manner; the material of the inner pressing ring is conductive, the ring body has a second conductive hole and a side hole, the positive power cable comprises a first positive power cable and a second positive power cable, one end of the first positive power cable is electrically connected to the plating region of the first side surface of the piezoelectric ceramic wafer, the other end of the first positive power cable is electrically connected to the side hole in a sealed manner, one end of the second positive power cable is electrically connected to the second conductive hole, and the other end of the second positive power cable passes through the cable hole.

In addition, further example provides an ultrasound focusing treatment head, the ultrasound focusing treatment head comprises a B-mode ultrasound probe and the ultrasound focusing transducer described above, and the B-mode ultrasound probe is accommodated in the installing hole and the spherical-segment shaped through hole.

Preferably, the B-mode ultrasound probe comprises an abdominal probe. Beneficial effect of the present invention

The ultrasound focusing transducer and the ultrasound focusing treatment head of the present examples press and fix the piezoelectric ceramic wafer in the shell by means of the fixed sealing part. The clamping part of the support presses and clamps the periphery of the first side opening of the piezoelectric ceramic wafer and fixes the piezoelectric ceramic wafer without using a gluing manner. The first side surface of the piezoelectric ceramic wafer, the support and the fixed sealing part form a sealed air cavity, which overcomes the adverse impact of the high-frequency vibration of the piezoelectric ceramic wafer on the sealing performance of the air cavity. The sealing performance of the air cavity is excellent, improving the stability of the product, and prolonging the service life of the product.

### BRIEF DESCRIPTION OF THE DRAWINGS

FIG. 1 a top view of an ultrasound focusing transducer according to an embodiment of the present invention;
FIG. 2 is a cross-sectional view of the ultrasound focusing transducer of FIG. 1 taken along the line A-A;
FIG. 3 is an exploded schematic view of a portion of the elements of the ultrasound focusing transducer in FIG. 2;
FIG. 4 is a top view of a shell shown in FIG. 2;
FIG. 5 is a cross-sectional view of the shell in FIG. 4 taken along line C-C;
FIG. 6 is a perspective schematic view of a piezoelectric ceramic wafer shown in FIG. 2;
FIG. 7 is a perspective schematic view of a connecting cylinder shown in FIG. 2;
FIG. 8 is a top view of the connecting cylinder of FIG. 7;
FIG. 9 is a cross-sectional view of the connecting cylinder of FIG. 8 taken along the line B-B;
FIG. 10 is a perspective schematic view of a core tube shown in FIG. 2;
FIG. 11 is a perspective schematic view of an inner pressing ring shown in FIG. 2;
FIG. 12 is a top view of the inner pressing ring in FIG. 11;
FIG 13 is a cross-sectional view of the inner pressing ring in FIG 12 taken along the line D-D;
FIG. 14 is a perspective schematic view of an annular baffle shown in FIG. 2;
FIG. 15 is a front view of a first clamping part or a second clamping part of an ultrasound focusing treatment head according to an embodiment of the present invention; and
FIG. 16 is a top view when the first clamping part and the second clamping part shown in FIG. 15 are installed on the connecting cylinder.

The realization of the objective, functional features and advantages of the present invention will be further described in conjunction with the embodiments and with reference to the accompanying drawings.

### DESCRIPTION OF THE EMBODIMENTS

It should be understood that the specific embodiments described herein are merely used to illustrate the present invention and are not intended to limit the present invention.

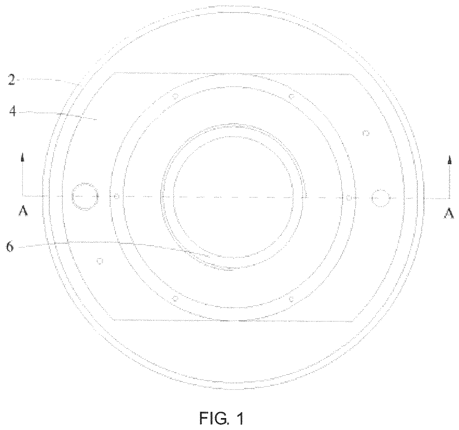

The present invention provides an ultrasound focusing transducer. Referring to FIGs. 1 to 3, in one embodiment, the ultrasound focusing transducer comprises a shell 2 and a piezoelectric ceramic wafer 8 accommodated in the shell 2, a fixed sealing part 5, a power cable 10, a waterproof cable head 1, a support 3 having an installing hole 30, and sealing liquid (not shown in the figures).

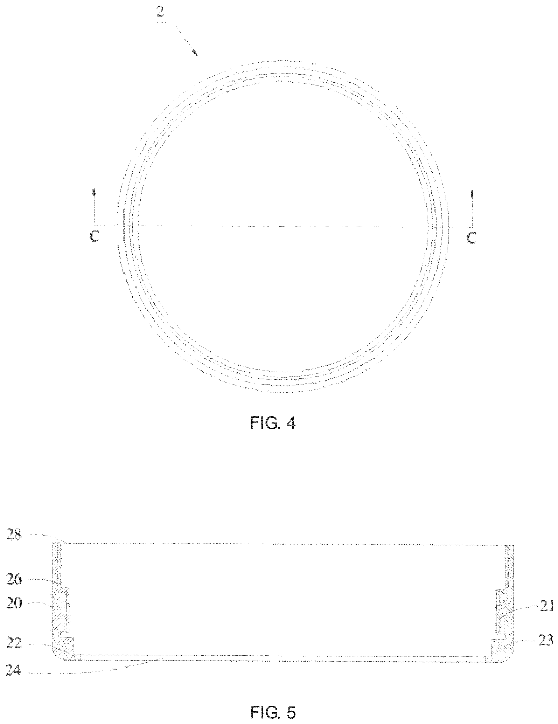

Referring to FIGs. 4 and 5, in the present embodiment, the shell 2 has a cylindrical shape, including a side wall 20 and an annular projection 22 extending radially inwardly from one end of the side wall 20. The cylindrical shell 2 forms two openings opposite to each other, one of which is a first opening 24 formed by the annular projection 22 and the other of which is a second opening 28 formed by the end of the side wall 20 away from the annular projection 22. An inner surface of the shell 2 connected to the annular projection 22 extends radially inwardly and forms a first step 23, and an inner surface of the shell 2 between the second opening 28 and the first step 23 extending radially inwardly and forms a second step 21. The size of a hole defined by the first step 23 is smaller than that of a hole defined by the second step 21, and the size of the hole defined by the annular projection 22 is smaller than that of the hole defined by the first step 23. In addition, a second internal thread 26 is formed on the inner surface of the shell 2, on the inner surface of the second step 21.

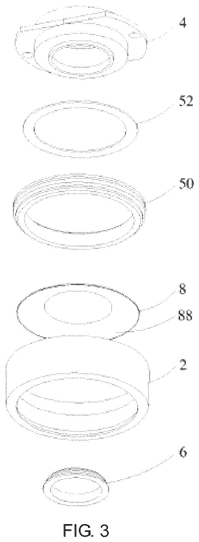

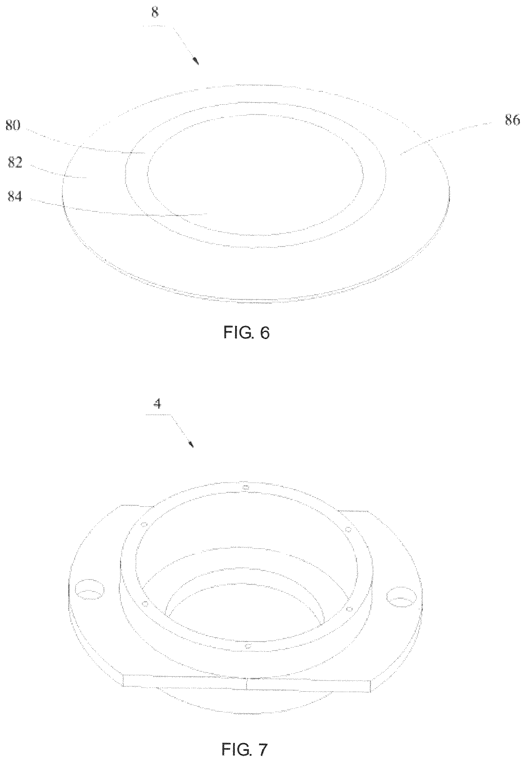

Referring to FIG. 6, in the present embodiment, the piezoelectric ceramic wafer 8 has a spherical-segment shape and is provided with a spherical-segment shaped through hole. The spherical-segment shaped through hole is provided with a first side opening 84 and a second side opening (not shown) which are both circular and arranged oppositely, wherein the hole size of the first side opening 84 is smaller than that of the second side opening. The piezoelectric ceramic wafer 8 protrudes toward the second opening 28. The piezoelectric ceramic wafer 8 has a first side surface 86 facing the second opening 28, and has a second side surface 88 facing the first opening 24 and back to the first side surface 86, as shown in FIG. 3. The hole size of the first side opening 84 is preferably 55 mm, and the hole size of the second side opening is preferably 115 mm.

Further, the piezoelectric ceramic wafer 8 is made of metasilicate ceramic, and includes a ceramic region 80 and a plating region 82, wherein the ceramic region 80 is a region of the piezoelectric ceramic wafer 8 without any metal plated on the surface. Specifically, the ceramic region 80 is annular and arranged on the first side surface 86 of the piezoelectric ceramic wafer 8 around the periphery of the first side opening 84, the plating region 82 is a region of the piezoelectric ceramic wafer 8 with a silver layer plated on the surface. Specifically, the plating region 82 is located in a region other than the ceramic region 80. The plating region 82 is adapted to be electrically connected to the power cable 10.

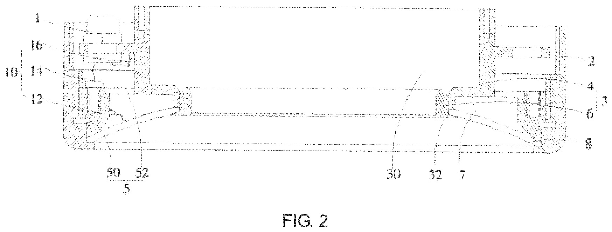

Referring to FIG. 2, in the present embodiment, the power cable 10 is electrically connected to the plating region 82 of the piezoelectric ceramic wafer 8. The power cable 10 includes a positive power cable and a negative power cable 16, wherein the positive power cable comprises a first positive power cable 12 and a second positive power cable 14. The second positive power cable 14 and the negative power cable 16 are connected to the waterproof cable head 1, providing a high-frequency voltage to the piezoelectric ceramic wafer 8 through the waterproof cable head 1 such that the piezoelectric ceramic wafer 8 vibrates and outputs ultrasonic waves of the same frequency.

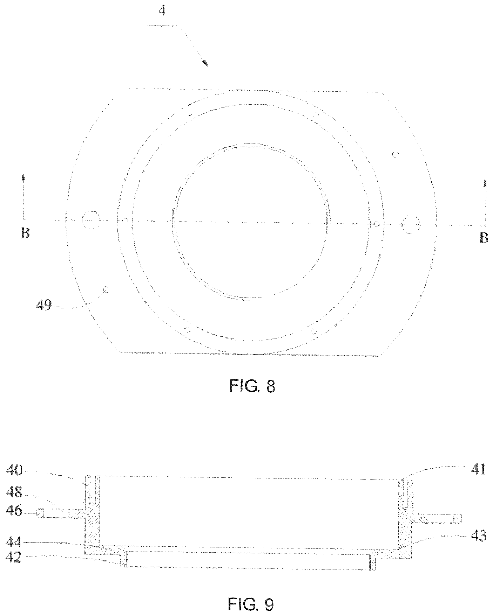

Referring to FIGs. 7 to 10, in the present embodiment, the material of the support 3 is conductive, preferably brass. The support 3 comprises a connecting cylinder 4 and a core tube 6. The connecting cylinder 4 and the core tube 6 are connected together to form a clamping part 32 and an installing hole 30. As shown in FIG. 2, the clamping part 32 is adapted to press and clamp the periphery of the first side opening 84 to fix the support 3 on the piezoelectric ceramic wafer 8. The installing hole 30 is aligned with the first side opening 84 of the piezoelectric ceramic wafer 8 for installing a B-mode ultrasound probe. The B-mode ultrasound probe can be a cardiac probe or an abdominal probe.

Further, the connecting cylinder 4 includes a first cylinder body 40, an annular connecting part 44, a second cylinder body 42, and an extending part 46. The first cylinder 40 comprises a first end 41 and a second end 43. The annular connecting part 44 is formed by extending radially inwardly from the second end 43, and the second cylinder 42 is formed by extending from an inner periphery of the annular connecting part 44 along a direction away from the second end 43. The extending part 46 comprises two symmetrical fan-shaped piece. The extending part 46 extends outwardly from the outer surface of the first cylinder 40. The extending part 46 has a cable hole 48 and a first conductive hole 49, and the waterproof cable head 1 is installed in the cable hole 48. In addition, a first internal thread (not labeled) is formed on the inner surface of the second cylinder 42, as shown in FIGs. 7 to 9.

The inner diameter of the first cylinder 40 of the connecting cylinder 4 is preferably 75 mm, and the inner diameter of the second cylinder 42 is preferably 55 mm. The annular connecting part 44 extends radially outwardly with respect to the side wall of the second cylinder 42. The first cylinder 40 is connected to the annular connecting part 44, so as to increase the accommodating space of the connecting cylinder 4 with respect to the second cylinder 42. That is, the first cylinder 40 and the annular connecting part 44 forms a larger accommodating space so that the accommodating space can accommodate various types of B-mode ultrasound probe. That is, an abdominal probe with a higher resolution and a larger volume can be used to monitor the ultrasound treatment process. The B-mode ultrasound image transmitted in real time is clearer, improving the safety and accuracy of treatment, and thereby improving the treatment efficiency.

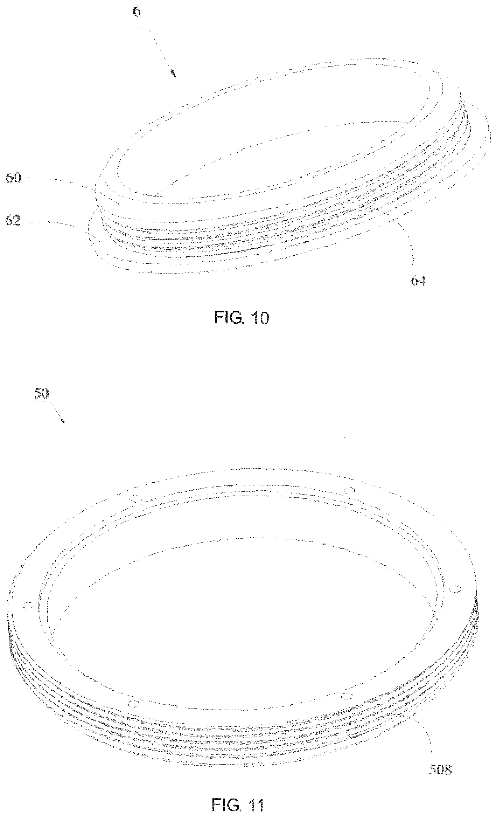

Further, the core tube 6 includes a tubular body 60 and a ring piece 62 extending radially outwardly from one end of the tubular body 60. The outer surface of the tube body 60 has a first external thread 64, as shown in FIG. 10.

The first internal thread of the inner surface of the second cylinder 42 is engaged with the first external thread 64 of the outer surface of the tube body 60. The clamping part 32 is formed between the end of the second cylinder 42 and the ring piece 62.

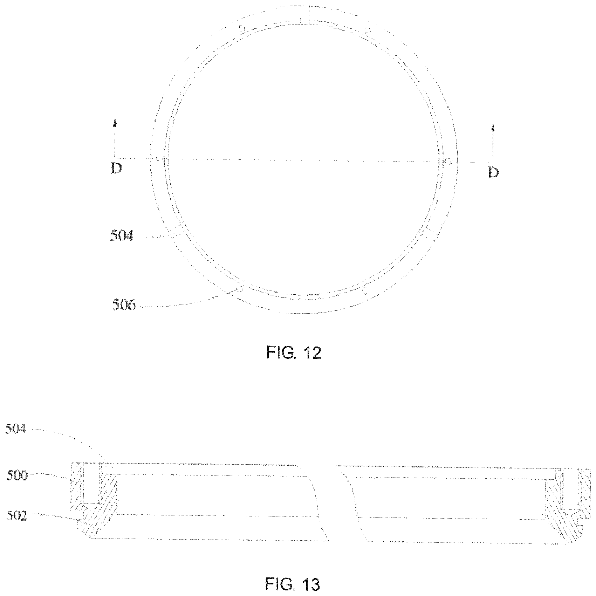

Referring to FIGs. 11 to 14, in the present embodiment, the fixed sealing part 5 includes an inner pressing ring 50 and an annular baffle 52. The inner pressing ring 50 includes a ring body 500 and an abutment part 502 extending axially from one end of the ring body 500. The abutment part 502 is adapted to abut against and press a first side surface 86 of the piezoelectric ceramic wafer 8. A recessed step 504 is provided on the inner periphery of the other end of the ring body 500. The annular baffle 52 is supported on the recess 504 and is fixedly connected to the inner pressing ring 20 in a sealed manner by gluing. A second external thread 508 is formed on the outer surface of the ring body 500.

Further, the material of inner pressing ring 50 is a conductive material, preferably brass. The ring body 500 has a second conductive hole 506 and a side hole 504. Referring to FIG. 2, one end of the first positive power cable 12 is electrically connected to the plating region 82 of the first side surface 86 of the piezoelectric ceramic wafer 8, the other end of the first positive power cable 12 is electrically connected to the side hole 504 in a sealed manner. One end of the second positive power cable 14 is electrically connected to the second conductive hole 506, and the other end of the second positive power cable 14 is connected to the waterproof cable head 1.

The assembling process of the ultrasound focusing transducer of the present embodiment is illustrated as follows:

S1: The abutment part 502 of the inner pressing ring 50 is fixed to the first side surface 86 of the piezoelectric ceramic wafer 8, wherein one end of the first positive power cable 12 is welded to the plating region 82 of the first side surface 86 of the piezoelectric ceramic wafer 8 and the other end of the first positive power cable 12 is fixed to the side hole 504 of the inner press ring 50 in a sealed manner. One end of the second positive power cable 14 is electrically connected to the second conductive hole 506 of the inner press ring 50.

S2: The inner presser ring 50 and the piezoelectric ceramic wafer 8 are integrally placed in the shell 2. The second external thread 508 of the inner pressing ring 50 is engaged with the second internal thread of the shell 2. The inner pressing ring 50 is screwed to the shell 2 so that one end of the piezoelectric ceramic wafer 8 forming the second side opening abuts against the joint between the annular projection 22 and the side wall 20 of the shell 2, and one end of the piezoelectric ceramic wafer 8 forming the first side opening 84 faces the other end of the shell 2. In the meantime, the abutment part 502 of the inner pressing ring 50 abuts against the first side surface 86 of the piezoelectric ceramic wafer 8 so as to fix the inner pressing ring 50 and the piezoelectric ceramic wafer 8 in the shell 2.

S3: The annular baffle 52 is supported on the recessed step 504 of the ring body 500 of the inner pressing ring 50 and is fixedly connected thereto in a sealed manner by gluing.

S4: The end of the second cylinder 42 of the connecting cylinder 4 passes through the annular baffle 52 and abuts against the ceramic region 80 of the piezoelectric ceramic wafer 8. The core tube 6 is nested into the second cylinder 42 of the connecting cylinder 4 from the second side surface 88 of the piezoelectric ceramic wafer 8, wherein the first external thread 64 of the outer surface of the tube body 60 of the core tube 6 is engaged with the first internal thread of the inner surface of the second cylinder 42 so that the tube body 60 is screwed into and tightened to the second cylinder 42. The clamping part 32 is formed between the ring piece 62 of the core tube 6 and the end of the second cylinder 42. The clamping part 32 presses and clamps the periphery of the first side opening 84 of the piezoelectric ceramic wafer 8. The annular baffle 52 is fixedly connected to the first cylinder 40 in a sealed manner by gluing. In the ultrasound focusing transducer of the this embodiment, the piezoelectric ceramic wafer 8 is fixed in the shell 2 by the fixed sealing part 5, and the clamping part 32 of the support 3 presses and clamps the periphery of the first side opening of the piezoelectric ceramic wafer 8, and thus fixing the piezoelectric ceramic wafer 8 without using glue. Thus, the piezoelectric ceramic wafer 8, the support 3 and the fixed sealing part 5 form a sealed air cavity 7, which overcomes the influence of the high-frequency vibration of the piezoelectric ceramic wafer 8 on the sealing performance of the air cavity 7. The sealing performance of the air cavity 7 is excellent, improving the stability of the product, and prolonging the service life of the ultrasound focusing transducer.

S5: One end of the negative power cable 16 is electrically connected to the first conductive hole 49 of the extending part 46 of the connecting cylinder 4, and the other end of the negative power cable 16 is connected to the waterproof cable head 1 mounted in the cable hole 48. The other end of the second positive power cable 14 is connected to the waterproof cable head 1. The negative power cable 16 connected to the waterproof cable head 1 is electrically connected to the extending part 46 of the first cylinder 40. The second cylinder 42 is electrically connected to the core tube 6. The ring piece 62 of the core tube 6 is electrically connected to the second side surface 88 of the piezoelectric ceramic wafer 8, and the end of the second cylinder 42 abuts against the insulating ceramic region 80. Therefore, the ring piece 62 of the core tube 6 is indirectly connected to the negative power cable 16 through the connecting cylinder 4, functioning as a negative electrode to provide power to the piezoelectric ceramic wafer 8 without additional cables passing through the air cavity 7, and preventing the sealing performance of the air cavity 7 from being influenced.

S6: Sealing liquid is injected into the shell 2 from an opening formed between the extending part 46 and the shell 2 and is filled in a gap formed between the shell 2, the support 3 and the fixed sealing part 5. The sealing liquid solidifies to further improve the sealing and waterproof performance.

The operation principle of the ultrasound focusing transducer of the present embodiment is illustrated as follows. The end of the first positive power cable 12 welded on the plating region 82 of the first side surface 86 of the piezoelectric ceramic wafer 8 acts as a positive electrode, and the other end of the first positive power cable 12 is electrically connected to the inner pressing ring 50 in a sealed manner. The inner pressing ring 50, as a conductor, is electrically connected to one end of the second positive electrode cable 14. The ring piece 62 of the core tube 6 acts as a negative electrode. The connecting cylinder 4, as a conductor, is electrically connected to one end of the negative power cable 16. Thus, the positive and negative electrodes provide a high-frequency voltage to the piezoelectric ceramic wafer 8 through an indirectly connected waterproof cable head 1. The piezoelectric ceramic wafer 8 converts the high-frequency voltage into ultrasonic waves of the same frequency.

The present invention further provides an ultrasound focusing treatment head. In the present embodiment, the ultrasound focusing treatment head includes a B-mode ultrasound probe and the ultrasound focusing transducer described above, and the B-mode ultrasound probe is accommodate in the installing hole 30 and the spherical-segment shaped through hole.

In the present embodiment, the B-mode ultrasound probe includes an abdominal probe.

The ultrasound focusing treatment head has an improved stability and prolonged service life due to the excellent sealing performance of the air cavity of the ultrasound focusing transducer. In the meantime, an abdominal probe with a higher resolution and a larger volume is allowed to be used to monitor the ultrasound treatment process. The B-mode ultrasound image transmitted in real time is clearer, improving the safety and accuracy of the treatment, and thereby improving the treatment efficiency of the ultrasound focusing treatment head.

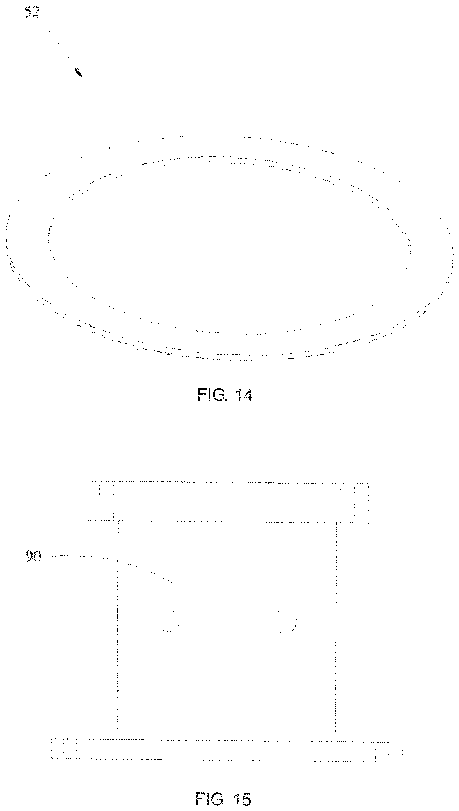

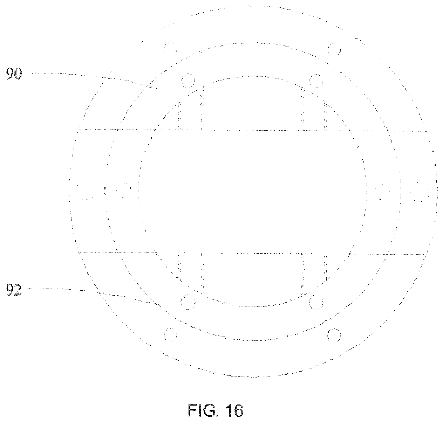

Referring to FIG. 15 and FIG. 16, in the present embodiment, the ultrasound focusing treatment head further comprises a first clamping member 90 and a second clamping member 92. The first clamping member 90 and the second clamping member 92 have the same structure. The first clamping member 90 and the second clamping member 92 are arranged opposite to each other, and are fixed to the first end 41 of the first cylinder 40 of the connecting cylinder 4 of the support 3. The first clamping part 90 and the second clamping part 92 cooperatively clamp the B-mode ultrasound probe. When the B-mode ultrasound probe is accommodated in the installing hole 30 of the support 3, the first clamping part 90 is engaged with the second clamping part 92 to cooperatively fix and clamp the portion of the B-mode ultrasound probe extends out of the connecting cylinder 4 of the support 3, such that monitoring center of the B-mode ultrasound probe can be aligned with the focus of the piezoelectric ceramic wafer, thus fixedly installing the B-mode ultrasound probe in the ultrasound focusing transducer on the premise of accurately positioning.

Further, the first clamping member 90 has a first clamping surface, and the second clamping member 92 has a second clamping surface. The first clamping surface is parallel to the second clamping surface and is in a shape of "I". The first clamping surface is engaged with the second clamping surface for clamping the B-mode ultrasound probe. Specifically, the distance between the first clamping surface and the second clamping surface is greater than zero and smaller than the inner diameter of the first cylinder 40, wherein the inner diameter of the first cylinder 40 is preferably 75 mm.

By adjusting the distance between the first clamping surface and the second clamping surface, B-mode ultrasound probes of different sizes can be clamped. When the B-mode ultrasound probe is a cardiac probe, the distance between the first clamping surface and the second clamping surface is small, and when the B-mode ultrasound probe is an abdominal probe, the distance between the first clamping surface and the second clamping surface is large.

The foregoing is merely a preferred embodiment of the present invention and is not intended to limit the patent scope of the present invention.

## Claims

1. An ultrasound focusing transducer comprising a shell (2), and a piezoelectric ceramic wafer (8), a fixed sealing part (5), and a support (3) having an installing hole (30) accommodated in the shell (2);
the piezoelectric ceramic wafer (8) is of a spherical segment shape and has a spherical-segment shaped through hole which is provided with a first side opening (84) and a second side opening which are circular and arranged oppositely, wherein the hole size of the
first side opening (84) is smaller than that of the second side opening, one end of the piezoelectric ceramic wafer (8) forming the second side opening abuts against one end of the shell (2), and one end of the piezoelectric ceramic wafer (8) forming the first side opening (84) faces the other end of the shell (2);
the fixed sealing part (5) is annular and installed on the inner surface of the shell (2);
the support (3) comprises a clamping part (32), the clamping part (32) being adapted to press and clamp a periphery of the first side opening (84) of the piezoelectric ceramic wafer (8) and fix the support (3) on the piezoelectric ceramic wafer (8), and the installing hole (30) being communicated with the spherical-segment shaped through hole;
the shell (2) comprises a cylindrical side wall (20) and a projection (22) extending radially inwardly from one end of the side wall (20);
the fixed sealing part (5), the support (3) and the piezoelectric ceramic wafer (8) cooperatively form a sealed air cavity **characterised in that**
the fixed sealing part (5) abuts against the piezoelectric ceramic wafer (8), the end of the piezoelectric ceramic wafer (8) forming the second side opening abuts against a joint between the projection (22) and the side wall (20) of the shell (2), and the fixed sealing part (5) is connected to the support (3) in a sealed manner.

2. The ultrasound focusing transducer according to claim 1, **characterized in that**, the support (3) comprises a connecting cylinder (4) and a core tube (6), an inner surface of one end of the connecting cylinder (4) is mounted on an outer surface of one end of the core tube (6), and the clamping part (32) is formed between the core tube (6) and the connecting cylinder (4).

3. The ultrasound focusing transducer according to claim 2, **characterized in that**, the connecting cylinder (4) comprises a first cylinder (40), an annular connecting part (44), and a second cylinder (42), the first cylinder (40) has a first end (41) and a second end (43), the annular connecting part (44) is formed by extending radially inwardly from the second end (43), the second cylinder (42) is formed by extending from an inner periphery of the annular connecting part (44) along a direction away from the second end (43); the core tube (6) comprises a tubular body (60) and a ring piece (62) extending radially outwardly from one end of the tubular body (60); an inner surface of the second cylinder (42) is mounted on an outer surface of the tubular body (60), and the clamping part (32) is formed between an end of the second cylinder (42) and the ring piece (62).

4. The ultrasound focusing transducer according to claim 3, **characterized in that**, a first internal thread is formed on an inner surface of the second cylinder (42), a first external thread (64) is formed on an outer surface of the tubular body (60), and the second cylinder (42) is connected to the core tube (6) in a sealed manner by engagement of the first internal thread with the first external thread (64).

5. The ultrasound focusing transducer according to claim 3, **characterized in that**, the piezoelectric ceramic wafer (8) has a protruded first side surface (86) and a recessed second side surface (88), the piezoelectric ceramic wafer (8) comprises a ceramic region (80) and a plating region (82) other than the ceramic region (80), the ceramic region (80) is annular and is arranged on the first side surface (86) around a periphery of the first side opening (84); material of the support (3) is conductive, an end of the second cylinder (42) abuts against the ceramic region (80), and the ring piece (62) acting as a negative electrode abuts against the plating region (82) of the second side surface (88).

6. The ultrasound focusing transducer according to claim 5, **characterized in that**, the ultrasound focusing transducer further comprises a power cable (10) electrically connected to the plating region (82) of the piezoelectric ceramic wafer (8), the power cable (10) comprises a negative power cable (16) and a positive power cable, the connecting cylinder (4) further comprises an extending part (46) arranged on an outer surface of the first cylinder (40), the extending part (46) has a cable hole (48) and a first conductive hole (49), one end of the negative power cable (16) is electrically connected to the first conductive hole (49), and the other end of the negative power cable (16) passes through the cable hole (48).

7. The ultrasound focusing transducer according to claim 6, **characterized in that**, the fixed sealing part (5) further comprises an inner pressing ring (50) and an annular baffle (52), the inner pressing ring (50) comprises a ring body (500) and an abutment part (502) extending axially from one end of the ring body (500), an outer surface of the ring body (500) is fixedly connected to an inner surface of the shell (2), the annular baffle (52) is fixed to an inner periphery of the ring body (500) in a sealed manner, and the abutment part (502) abuts against the first side surface (86) of the piezoelectric ceramic wafer (8).

8. The ultrasound focusing transducer according to claim 7, **characterized in that**, a second external thread (508) is formed on an outer surface of the ring body (500), and a second internal thread (26) is formed on an inner surface of the shell (2), the inner pressing ring (50) and the shell (2) are fixedly connected by engagement of the second external thread (508) and the second internal thread (26), an inner surface of the annular baffle (52) and an outer surface of the first cylinder (40) are in contact with each other, and fixedly connected together by gluing in a sealed manner; the material of the inner pressing ring (50) is conductive, the ring body (500) has a second conductive hole (506) and a side hole (504), the positive power cable comprises a first positive power cable (12) and a second positive power cable (14), one end of the first positive power cable (12) is electrically connected to the plating region (82) of the first side surface (86) of the piezoelectric ceramic wafer (8), the other end of the first positive power cable (12) is electrically connected to the side hole (504) in a sealed manner, one end of the second positive power cable (14) is electrically connected to the second conductive hole (506), and the other end of the second positive power cable (14) passes through the cable hole (48).

9. An ultrasound focusing treatment head, **characterized in that**, the ultrasound focusing treatment head comprises a B-mode ultrasound probe and the ultrasound focusing transducer according to any one of claims 1 to 8, and the B-mode ultrasound probe is accommodate in the installing hole (30) and the spherical-segment shaped through hole.

10. The ultrasound focusing treatment head according to claim 9, **characterized in that**, the B-mode ultrasound probe comprises an abdominal probe.

## Patentansprüche

1. Ultraschall-Fokussierwandler mit einer Schale (2) und einem piezoelektrischen Keramikplättchen (8), einem feststehenden Dichtungsteil (5) und einem Träger (3) mit einem in der Schale (2) untergebrachten Einbauloch (30) ;
der piezoelektrische Keramikwafer (8) kugelsegmentförmig ist und ein kugelsegmentförmiges Durchgangsloch aufweist, das mit einer ersten Seitenöffnung (84) und einer zweiten Seitenöffnung versehen ist, die kreisförmig und gegenüberliegend angeordnet sind, wobei die Lochgröße der ersten Seitenöffnung (84) kleiner ist als die der zweiten Seitenöffnung, ein Ende des die zweite Seitenöffnung bildenden piezoelektrischen Keramikwafers (8) an einem Ende der Schale (2) anliegt, und ein Ende des die erste Seitenöffnung (84) bildenden piezoelektrischen Keramikwafers (8) dem anderen Ende der Schale (2) gegenüberliegt;
das feststehende Dichtungsteil (5) ist ringförmig und an der Innenfläche der Schale (2) angebracht;
der Träger (3) umfasst ein Klemmteil (32), wobei das Klemmteil (32) angepasst ist, um einen Umfang der ersten Seitenöffnung (84) des piezoelektrischen Keramikwafers (8) zu drücken und zu klemmen und den Träger (3) auf dem piezoelektrischen Keramikwafer (8) zu fixieren, und wobei das Installationsloch (30) mit dem kugelsegmentförmigen Durchgangsloch verbunden ist;
das Gehäuse (2) eine zylindrische Seitenwand (20) und einen Vorsprung (22) umfasst, der sich von einem Ende der Seitenwand (20) radial nach innen erstreckt;
das feste Dichtungsteil (5), der Träger (3) und der piezoelektrische Keramikwafer (8) zusammen einen abgedichteten Lufthohlraum bilden, **dadurch gekennzeichnet, dass**
das feste Dichtungsteil (5) am piezoelektrischen Keramikwafer (8) anliegt, das die zweite Seitenöffnung bildende Ende des piezoelektrischen Keramikwafers (8) an einer Fuge zwischen dem Vorsprung (22) und der Seitenwand (20) des Gehäuses (2) anliegt und das feste Dichtungsteil (5) mit dem Träger (3) dicht verbunden ist.

2. Ultraschall-Fokussierwandler nach Anspruch 1, **dadurch gekennzeichnet, dass** der Träger (3) einen Verbindungszylinder (4) und ein Kernrohr (6) umfasst, eine Innenfläche eines Endes des Verbindungszylinders (4) an einer Außenfläche eines Endes des Kernrohrs (6) angebracht ist und das Klemmteil (32) zwischen dem Kernrohr (6) und dem Verbindungszylinder (4) ausgebildet ist.

3. Ultraschall-Fokussierwandler nach Anspruch 2, **dadurch gekennzeichnet, dass** der Verbindungszylinder (4) einen ersten Zylinder (40), ein ringförmiges Verbindungsteil (44) und einen zweiten Zylinder (42) umfasst, der erste Zylinder (40) ein erstes Ende (41) und ein zweites Ende (43) aufweist, das ringförmige Verbindungsteil (44) dadurch gebildet ist, dass es sich von dem zweiten Ende (43) radial nach innen erstreckt, der zweite Zylinder (42) dadurch gebildet ist, dass er sich von einem inneren Umfang des ringförmigen Verbindungsteils (44) entlang einer Richtung weg vom zweiten Ende (43) erstreckt; das Kernrohr (6) einen röhrenförmigen Körper (60) und ein Ringstück (62) umfasst, das sich von einem Ende des röhrenförmigen Körpers (60) radial nach außen erstreckt; eine Innenfläche des zweiten Zylinders (42) an einer Außenfläche des röhrenförmigen Körpers (60) angebracht ist und das Klemmteil (32) zwischen einem Ende des zweiten Zylinders (42) und dem Ringstück (62) ausgebildet ist.

4. Ultraschall-Fokussierwandler nach Anspruch 3, **dadurch gekennzeichnet, dass** ein erstes Innengewinde an einer Innenfläche des zweiten Zylinders (42) ausgebildet ist, ein erstes Außengewinde (64) an einer Außenfläche des rohrförmigen Körpers (60) ausgebildet ist und der zweite Zylinder (42) durch Eingriff des ersten Innengewindes mit dem ersten Außengewinde (64) mit dem Kernrohr (6) dicht verbunden ist.

5. Ultraschall-Fokussierwandler nach Anspruch 3, **dadurch gekennzeichnet, dass** der piezoelektrische Keramikwafer (8) eine vorstehende erste Seitenfläche (86) und eine vertiefte zweite Seitenfläche (88) aufweist, der piezoelektrische Keramikwafer (8) einen Keramikbereich (80) und einen von dem Keramikbereich (80) verschiedenen Plattierungsbereich (82) umfasst, der Keramikbereich (80) ringförmig ist und auf der ersten Seitenfläche (86) um einen Umfang der ersten Seitenöffnung (84) angeordnet ist; Material des Trägers (3) leitfähig ist, ein Ende des zweiten Zylinders (42) an dem Keramikbereich (80) anliegt und das als negative Elektrode wirkende Ringstück (62) an dem Plattierungsbereich (82) der zweiten Seitenfläche (88) anliegt.

6. Ultraschall-Fokussierwandler nach Anspruch 5, **dadurch gekennzeichnet, dass** der Ultraschall-Fokussierwandler weiterhin ein Stromkabel (10) umfasst, das elektrisch mit dem Plattierungsbereich (82) des piezoelektrischen Keramikwafers (8) verbunden ist, wobei das Stromkabel (10) ein negatives Stromkabel (16) und ein positives Stromkabel umfasst, der Verbindungszylinder (4) ferner ein Verlängerungsteil (46) umfasst, das an einer Außenfläche des ersten Zylinders (40) angeordnet ist, das Verlängerungsteil (46) ein Kabelloch (48) und ein erstes leitfähiges Loch (49) aufweist, ein Ende des negativen Leistungskabels (16) elektrisch mit dem ersten leitfähigen Loch (49) verbunden ist und das andere Ende des negativen Leistungskabels (16) durch das Kabelloch (48) verläuft.

7. Ultraschall-Fokussierwandler nach Anspruch 6, **dadurch gekennzeichnet, dass** das feststehende Dichtungsteil (5) ferner einen inneren Pressring (50) und eine ringförmige Ablenkplatte (52) umfasst, wobei der innere Pressring (50) einen Ringkörper (500) und einen Anschlagteil (502) umfasst, der sich axial von einem Ende des Ringkörpers (500) erstreckt, eine äußere Oberfläche des Ringkörpers (500) fest mit einer inneren Oberfläche der Schale (2) verbunden ist, die ringförmige Ablenkplatte (52) an einem inneren Umfang des Ringkörpers (500) in einer abgedichteten Weise befestigt ist und das Anschlagteil (502) an der ersten Seitenoberfläche (86) des piezoelektrischen Keramikwafers (8) anliegt.

8. Ultraschall-Fokussierwandler nach Anspruch 7, **dadurch gekennzeichnet, dass** an einer Außenfläche des Ringkörpers (500) ein zweites Außengewinde (508) und an einer Innenfläche der Schale (2) ein zweites Innengewinde (26) ausgebildet ist, der innere Pressring (50) und die Schale (2) durch Eingriff des zweiten Außengewindes (508) und des zweiten Innengewindes (26) fest miteinander verbunden sind, eine Innenfläche der ringförmigen Ablenkplatte (52) und eine Außenfläche des ersten Zylinders (40) miteinander in Kontakt stehen und durch Verkleben dicht miteinander verbunden sind; das Material des inneren Pressrings (50) leitfähig ist, der Ringkörper (500) ein zweites leitfähiges Loch (506) und ein Seitenloch (504) aufweist, das positive Stromversorgungskabel ein erstes positives Stromversorgungskabel (12) und ein zweites positives Leistungskabel (14) umfasst, wobei ein Ende des ersten positiven Leistungskabels (12) elektrisch mit dem Plattierungsbereich (82) der ersten Seitenfläche (86) des piezoelektrischen Keramikwafers (8) verbunden ist, das andere Ende des ersten positiven Leistungskabels (12) mit dem Seitenloch (504) elektrisch dicht verbunden ist, ein Ende des zweiten positiven Leistungskabels (14) mit dem zweiten leitfähigen Loch (506) elektrisch verbunden ist und das andere Ende des zweiten positiven Leistungskabels (14) durch das Kabelloch (48) geführt wird.

9. Ultraschallfokussierungs-Behandlungskopf, **dadurch gekennzeichnet, dass** der Ultraschallfokussierungs-Behandlungskopf eine B-Mode-Ultraschallsonde und den Ultraschallfokussierungswandler nach einem der Ansprüche 1 bis 8 umfasst, und die B-Mode-Ultraschallsonde in der Installationsöffnung (30) und der kugelsegmentförmigen Durchgangsöffnung untergebracht ist.

10. Ultraschallfokussierungs-Behandlungskopf nach Anspruch 9, **dadurch gekennzeichnet, dass** die B-Mode-Ultraschallsonde eine Abdomensonde umfasst.

## Revendications

1. Transducteur de focalisation d'ultrasons comprenant une coque (2), et une plaquette céramique piézoélectrique (8), une partie d'étanchéité fixe (5) et un support (3) présentant un trou d'installation (30) logé dans la coque (2) ;
la plaquette céramique piézoélectrique (8) est d'une forme de segment sphérique et présente un trou traversant en forme de segment sphérique qui est pourvu d'une première ouverture latérale (84) et d'une seconde ouverture latérale qui sont circulaires et agencées de façon opposée, dans lequel la taille de trou de la première ouverture latérale (84) est plus petite que celle de la seconde ouverture latérale, une extrémité de la plaquette céramique piézoélectrique (8) formant la seconde ouverture latérale vient buter contre une extrémité de la coque (2), et une extrémité de la plaquette céramique piézoélectrique (8) formant la première ouverture latérale (84) fait face à l'autre extrémité de la coque (2) ;
la partie d'étanchéité fixe (5) est annulaire et installée sur la surface intérieure de la coque (2) ;
le support (3) comprend une partie de serrage (32), la partie de serrage (32) étant adaptée pour presser et serrer une périphérie de la première ouverture latérale (84) de la plaquette céramique piézoélectrique (8) et fixer le support (3) sur la plaquette céramique piézoélectrique (8), et le trou d'installation (30) communiquant avec le trou traversant en forme de segment sphérique ;
la coque (2) comprend une paroi latérale cylindrique (20) et une saillie (22) s'étendant radialement vers l'intérieur depuis une extrémité de la paroi latérale (20) ;
la partie d'étanchéité fixe (5), le support (3) et la plaquette céramique piézoélectrique (8) forment de façon coopérative une cavité d'air étanche, **caractérisé en ce que** la partie d'étanchéité fixe (5) vient buter contre la plaquette céramique piézoélectrique (8), l'extrémité de la plaquette céramique piézoélectrique (8) formant la seconde ouverture latérale vient buter contre un raccord entre la saillie (22) et la paroi latérale (20) de la coque (2), et la partie d'étanchéité fixe (5) est reliée au support (3) de manière étanche.

2. Transducteur de focalisation d'ultrasons selon la revendication 1, **caractérisé en ce que** le support (3) comprend un cylindre de liaison (4) et un tube central (6), une surface intérieure d'une extrémité du cylindre de liaison (4) est montée sur une surface extérieure d'une extrémité du tube central (6), et la partie de serrage (32) est formée entre le tube central (6) et le cylindre de liaison (4).

3. Transducteur de focalisation d'ultrasons selon la revendication 2, **caractérisé en ce que** le cylindre de liaison (4) comprend un premier cylindre (40), une partie de liaison annulaire (44) et un second cylindre (42), le premier cylindre (40) présente une première extrémité (41) et une seconde extrémité (43), la partie de liaison annulaire (44) est formée en s'étendant radialement vers l'intérieur depuis la seconde extrémité (43), le second cylindre (42) est formé en s'étendant depuis une périphérie intérieure de la partie de liaison annulaire (44) le long d'une direction opposée à la seconde extrémité (43) ; le tube central (6) comprend un corps tubulaire (60) et une pièce annulaire (62) s'étendant radialement vers l'extérieur depuis une extrémité du corps tubulaire (60) ; une surface intérieure du second cylindre (42) est montée sur une surface extérieure du corps tubulaire (60), et la partie de serrage (32) est formée entre une extrémité du second cylindre (42) et la pièce annulaire (62).

4. Transducteur de focalisation d'ultrasons selon la revendication 3, **caractérisé en ce qu'**un premier filetage interne est formé sur une surface intérieure du second cylindre (42), un premier filetage externe (64) est formé sur une surface extérieure du corps tubulaire (60), et le second cylindre (42) est relié au tube central (6) de manière étanche par mise en prise du premier filetage interne avec le premier filetage externe (64).

5. Transducteur de focalisation d'ultrasons selon la revendication 3, **caractérisé en ce que** la plaquette céramique piézoélectrique (8) présente une première surface latérale en saillie (86) et une seconde surface latérale évidée (88), la plaquette céramique piézoélectrique (8) comprend une région céramique (80) et une région de placage (82) autre que la région céramique (80), la région céramique (80) est annulaire et est agencée sur la première surface latérale (86) autour d'une périphérie de la première ouverture latérale (84) ; le matériau du support (3) est conducteur, une extrémité du second cylindre (42) vient buter contre la région céramique (80), et la pièce annulaire (62) servant d'électrode négative vient buter contre la région de placage (82) de la seconde surface latérale (88).

6. Transducteur de focalisation d'ultrasons selon la revendication 5, **caractérisé en ce que** le transducteur de focalisation d'ultrasons comprend en outre un câble d'alimentation (10) électriquement relié à la région de placage (82) de la plaquette céramique piézoélectrique (8), le câble d'alimentation (10) comprend un câble d'alimentation négatif (16) et un câble d'alimentation positif, le cylindre de liaison (4) comprend en outre une partie d'extension (46) agencée sur une surface extérieure du premier cylindre (40), la partie d'extension (46) présente un trou pour câble (48) et un premier trou conducteur (49), une extrémité du câble d'alimentation négatif (16) est électriquement reliée au premier trou conducteur (49), et l'autre extrémité du câble d'alimentation négatif (16) traverse le trou pour câble (48).

7. Transducteur de focalisation d'ultrasons selon la revendication 6, **caractérisé en ce que** la partie d'étanchéité fixe (5) comprend en outre un anneau de pressage intérieur (50) et un déflecteur annulaire (52), l'anneau de pressage intérieur (50) comprend un corps d'anneau (500) et une partie de butée (502) s'étendant axialement depuis une extrémité du corps d'anneau (500), une surface extérieure du corps d'anneau (500) est reliée de manière fixe à une surface intérieure de la coque (2), le déflecteur annulaire (52) est fixé à une périphérie intérieure du corps d'anneau (500) de manière étanche, et la partie de butée (502) vient buter contre la première surface latérale (86) de la plaquette céramique piézoélectrique (8).

8. Transducteur de focalisation d'ultrasons selon la revendication 7, **caractérisé en ce qu'**un second filetage externe (508) est formé sur une surface extérieure du corps d'anneau (500), et un second filetage interne (26) est formé sur une surface intérieure de la coque (2), l'anneau de pressage intérieur (50) et la coque (2) sont reliés de manière fixe par mise en prise du second filetage externe (508) et du second filetage interne (26), une surface intérieure du déflecteur annulaire (52) et une surface extérieure du premier cylindre (40) sont en contact l'une avec l'autre, et reliées de manière fixe ensemble par collage de manière étanche ; le matériau de l'anneau de pressage intérieur (50) est conducteur, le corps d'anneau (500) présente un second trou conducteur (506) et un trou latéral (504), le câble d'alimentation positif comprend un premier câble d'alimentation positif (12) et un second câble d'alimentation positif (14), une extrémité du premier câble d'alimentation positif (12) est électriquement reliée à la région de placage (82) de la première surface latérale (86) de la plaquette céramique piézoélectrique (8), l'autre extrémité du premier câble d'alimentation positif (12) est électriquement reliée au trou latéral (504) de manière étanche, une extrémité du second câble d'alimentation positif (14) est électriquement reliée au second trou conducteur (506), et l'autre extrémité du second câble d'alimentation positif (14) traverse le trou pour câble (48).

9. Tête de traitement de focalisation d'ultrasons, **caractérisée en ce que** la tête de traitement de focalisation d'ultrasons comprend une sonde à ultrasons de mode B et le transducteur de focalisation d'ultrasons selon l'une quelconque des revendications 1 à 8, et la sonde à ultrasons de mode B est logée dans le trou d'installation (30) et le trou traversant en forme de segment sphérique.

10. Tête de traitement de focalisation d'ultrasons selon la revendication 9, **caractérisée en ce que** la sonde à ultrasons de mode B comprend une sonde abdominale.
